# EUROPÄISCHE PATENTSCHRIFT

(11) **EP 1 465 848 B1**
(45) Veröffentlichungstag und Bekanntmachung des Hinweises auf die Patenterteilung: **14.08.2013**
(21) Anmeldenummer: 02800003.2
(22) Anmeldetag: 30.08.2002
(51) Int. Cl.: C07C 1/00, C07C 67/303, C07C 69/75, C08K 5/12

(54) **GEMISCH ALICYCLISCHER POLYCARBONSÄUREESTER MIT HOHEM CIS-ANTEIL**
MIXTURE OF ALICYCLIC POLYCARBOXYLIC ACID ESTERS HAVING A HIGH CIS ISOMER CONTENT
MELANGE D'ESTERS D'ACIDE POLYCARBOXYLIQUE ALICYCLIQUES A HAUTE TENEUR EN ISOMERES CIS

(30) Priorität: 24.09.2001 DE 10146848
(43) Veröffentlichungstag der Anmeldung: 13.10.2004
(73) Patentinhaber: Evonik Oxeno GmbH, 45772 Marl (DE)
(72) Erfinder: BÜSCHKEN, Wilfried, 45721 Haltern am See (DE); GRASS, Michael, 45721 Haltern am See (DE); KAIZIK, Alfred, 45772 Marl (DE); MASCHMEYER, Dietrich, 45657 Recklinghausen (DE); TUCHLENSKI, Axel, 45478 Mülheim (DE); NIERLICH, Franz, 45768 Marl (DE)
(74) Vertreter: Hirsch, Hans-Ludwig
(86) Internationale Anmeldenummer: PCT/EP2002/009732
(87) Internationale Veröffentlichungsnummer: WO 2003/029168

(56) Entgegenhaltungen:
- WO-A-00/78704
- S.SIEGEL ET AL.: "The stereochemistry of hydrogenaiton of isomers of methyl tetrahydrophthalate and methyl phthalate" J.AM.CHEM.SOC, Bd. 81, 1959, Seiten 3655-3658, XP009003698 in der Anmeldung erwähnt

## Beschreibung

Die vorliegende Erfindung betrifft Verfahren zur Herstellung von alicyclischen Polycarbonsäureester gemischen mit hohem cis-Anteil gemäss Anspruch 1.

Alicyclische Polycarbonsäureester, wie beispielsweise die Ester der Cyclohexan-1,2-dicarbonsäure, werden als Schmierölkomponente und als Hilfsmittel bei der Metallverarbeitung eingesetzt. Weiterhin finden sie als Weichmacher für Polyolefine Verwendung.

Für die Weichmachung von PVC werden zur Zeit überwiegend Ester der Phthalsäure, wie beispielweise Dibutyl- , Dioctyl-, Dinonyl- oder Didecylester, verwendet. Da diese Phthalate in letzter Zeit als gesundheitsschädlich bezeichnet werden, muss befürchtet werden, dass deren Einsatz in Kunststoffen eingeschränkt werden könnte. Alicyclische Polycarbonsäurester, von denen einige in der Literatur als Weichmacher für verschiedene Kunststoffe beschrieben sind, könnten dann als geeignete Ersatzstoffe, wenn auch mit einem etwas anderem anwendungstechnischen Profil, zur Verfügung stehen.

In den meisten Fällen ist der wirtschaftlichste Weg zur Herstellung von alicyclischen Polycarbonsäureestern die Kernhydrierung der entsprechenden aromatischen Polycarbonsäureester, beispielsweise der o. g. Phthalate. Es sind hierzu bereits einige Verfahren bekannt:

In US 5 286 898 und US 5 319 129 werden Verfahren beschrieben, mit dem Dimethylterephthalate an geträgerten Pd-Katalysatoren, die mit Ni, Pt und/oder Ru dotiert sind, bei Temperaturen größer oder gleich 140 °C und einem Druck zwischen 50 und 170 bar zum entsprechenden Hexahydrodimethylterephthalat hydriert werden kann.

In DE 28 23 165 werden aromatische Carbonsäureester an geträgerten Ni, Ru, Rh und/oder Pd-Katalysatoren zu den entsprechenden alicyclischen Carbonsäureestern bei 70 bis 250 °C und 30 bis 200 bar hydriert. US 3 027 398 offenbart die Hydrierung von Dimethylterephthalat an geträgerten Ru-Katalysatoren bei 110 bis 140°C und 35 bis 105 bar.

In WO 00/78704 wird ein Verfahren zur Hydrierung von Benzolpolycarbonsäureester zu den entsprechenden alicyclischen Verbindungen offengelegt. Dabei werden Trägerkatalysatoren eingesetzt, die ein Metall der VIII. Nebengruppe alleine oder zusammen mit mindestens einem Metall der I. oder VII. Nebengruppe des Periodensystems enthalten und 50% Makroporen aufweisen. Als bevorzugtes Metall der VIII. Nebengruppe wird Ruthenium eingesetzt.

Bei der Kernhydrierung von aromatischen Polycarbonsäureestern können bezüglich des Ringsystems und der Esterfunktionen mindestens zwei Isomere entstehen.

Beispielsweise sind bei der Hydrierung von Phthalsäurediester (1,2-Benzoldicarbonsäureester) cis- und/oder trans-Cyclohexan-1,2-dicarbonsäurediester die Produkte. Dabei versteht man unter cis-Diester dasjenige Isomer, bei dem die eine Estergruppe axial (a) und die andere äquatorial (e) ausgerichtet ist. Unter trans-Verbindung ist dasjenige Isosomer zu verstehen, bei dem beide Estergruppen entweder axial (a, a) oder äquatorial (e, e) ausgerichtet sind.

Bei der Hydrierung von Isophthalsäurediester (1,3-Benzoldicarbonsäurediester) können cis- und trans-1,3-Cydohexancarbonsäwediester entstehen. Bei der cis-Verbindung sind die Estergruppen entweder axial-axial (a, a) oder äquatorial-äquatorial (e, e) ausgerichtet. Bei der trans Verbindung ist eine Estergruppe axial und die andere äquatorial ausgerichtet.

Bei der Hydrierung von Terephthalsäurediester (1,4-Benzoldicarbonsäurediester) können, cis- und trans-1,4-Cyclohexandicarbonsäurediester entstehen. Dabei sind bei der cis-Verbindung eine Estergruppe axial und die andere äquatorial (a, e) ausgerichtet. Bei der trans-Verbindung sind beide Estergruppen entweder axial (a, a) oder äquatorial (e, e) ausgerichtet.

Bei alicyclischen Polycarbonsäureester mit mehr als zwei Substituenten am gleichen Ringsystem kann jeder Substituent zu einem anderen Substituenten cis- oder trans-konfiguriert sein. Im Sinne der vorliegenden Erfindung sind alle Verbindungen, bei denen die Mehrzahl der Estergruppen trans zueinander konfiguriert sind, unabhängig von den anderen Konfigurationen der Substituenten zueinander als trans-Verbindungen anzusehen.

Über die Konfiguration der Produkte, die bei der Kernhydrierung von aromatischen Polycarbonsäureestern entstehen, findet man in der Literatur nur wenige und lückenhafte Informationen.

Beispielweise entsteht nach US 3 027 165 bei der Hydrierung von Dimethyltherephthalat an einem Rutheniumkatalysator ein Gemisch aus cis- und trans-1,4-Cyclohexandicarbonsäuredimethylester mit einem Schmelzpunkt von unter 20 °C. Es ist bekannt, dass der Schmelzpunkt des trans-Diesters 70 °C und der des cis-Diesters 7 °C beträgt. Bei Annahme des üblichen Schmelzverhalten (keine Mischkristallbildung), d. h., dass ausgehend von einem reinen Isomeren bei Zugabe des anderen Isomeren der Schmelzpunkt des Gemisches so lange sinkt, bis der eutektische Punkt erreicht ist, kann abgeschätzt werden, dass das Hydriergemisch hauptsächlich aus cis-1,4-Cyclohexandicarbonsäuredimethylester besteht.

S. Siegel und G. McCaleb in JACS, 81, 1959, S. 3655-3658 beschreiben die Hydrierung von Phthalsäuredimethylestern an suspendiertem Platinoxidpulver in Eisessig. Es wurden druck- und konzentrationsunabhängig Gemische der entsprechenden Cyclohexansäurederivate mit einem cis-Anteil von praktisch 100 Mol-% erhalten. Die Ausbeuten der isolierten Ester sind nicht genannt. Diese Hydriermethode hat einige Nachteile: Der Katalysator muss aus dem Hydriergemisch abgetrennt werden, wobei erfahrungsgemäß Katalysatorverluste unvermeidbar sind. Das verwendete Lösungsmittel Eisessig ist sehr korrosiv und erfordert daher Apparaturen aus hochwertigen Werkstoffen. Weiterhin muss der Eisessig, aus dem der Hydrieraustrag zu 80 bis 90 % besteht, vom Zielprodukt abgetrennt werden.

Die Herstellung von Cyclohexandicarbonsäuredimethylester mit einem hohen cis-Anteil ist daher bekannt. Es ist dieser Publikation jedoch nicht zu entnehmen, ob auch andere Carbonsäureester mit hohem cis-Anteil durch die publizierte oder eine andere Methode zugänglich sind.

Werden die in WO 00/78704 offenbarten Ruthenium-haltigen Katalysatoren zur Hydrierung von Düsononylphthalaten eingesetzt, wird ein Produktgemisch mit ca. 93 Mol-% cis- und entsprechend 7 Mol-% des trans-Isomers erhalten.

Alicyclische Polycarbonsäureestergemische mit einem Anteil des/der cis-Isomeren von über 93

Mol-% unter der Ausnahme von Cyclohexandicarbonsäuremethylester sind daher nicht bekannt.

Aufgabe der vorliegenden Erfindung war es daher, solche Gemische herzustellen und auf ihre Verwendung als Weichmacher für Kunststoffe zu prüfen.

Gemäß Anspruch 1 hergestellte Polycarbonsäureestergemische sind daher alicyclische Polycarbonsäureestergemische unter der Ausnahme von Cyclohexandicarbonsäuremethylester, enthaltend mindestens zwei Isomere bezüglich der Stellung der Estergruppen am Ringsystem, wobei der Anteil der cis-Isomere über 93 Mol-% beträgt.

Die Herstellung der erfindungsgemäßen Gemische erfolgt bevorzugt durch Hydrierung der entsprechenden aromatischen Polycarbonsäureester. Hierzu kann ein Katalysator eingesetzt werden, der mindestens ein Edelmetall aus der VIII. Nebengruppe der Elemente und mindestens ein Metall der II. Nebengruppe des Periodensystems enthält.

Ein weiterer Gegenstand der vorliegenden Erfindung ist ein Verfahren zur Herstellung von alicyclischen Polycarbonsäureestergemischen, die mindestens zwei Isomere bezüglich der Stellung der Estergruppen am Ringsystem und die einen Anteil des cis-Isomer über 93 Mol-% aufweisen, durch katalytische Hydrierung der entsprechenden aromatischen Polycarbonsäureester, wobei der Katalysator mindestens ein Edelmetall aus der VIII. Nebengruppe (Ruthenium, Rhodium, Palladium, Osmium, Iridium, Platin) und mindestens ein Metall der II. Nebengruppe des Periodensystems enthält.

Von den oben genannten Edelmetallen werden Ruthenium und ganz besonders Palladium bevorzugt. Als bevorzugtes Metall der II. Nebengruppe des Periodensystems wird Zink eingesetzt.

Neben den genannten Edelmetallen und Zink können alle im erfindungsgemäßen Verfahren eingesetzten Katalysatoren zusätzlich inerte Träger, die z. B. aus den Metallen Aluminium, Magnesium, Titan, Zirkonium und/oder Silicium, als Oxid oder Mischoxid bestehen, enthalten. Optional können die Katalysatoren auch Salze der genannten Trägermetalle, wie beispielsweise Sulfate und/oder Phosphate enthalten. Weiterhin können die erfindungsgemäß eingesetzten Katalysatoren Verarbeitungs- und Formgebungshilfsmittel wie beispielsweise Graphit beinhalten.

Die im Folgenden angegebenen Zusammensetzungen beziehen sich auf die reduzierten Katalysatoren.

Der Edelmetallgehalt der Katalysatoren (berechnet als Metall) liegt im Bereich von 0,1 bis 10 Massen-%, insbesondere im Bereich von 0,5 bis 5 Massen-%, ganz besonders zwischen 1 und 3 Massen-%.

Der Gehalt der Katalysatoren an Metallen der II. Nebengruppe z. B. Zink, (berechnet als Oxid) beträgt 3 bis 70 Massen-%, insbesondere 10 bis 50 Massen-%, ganz besonders 20, bis 30 Massen-%.

Im erfindungsgemäßen Verfahren werden besonders bevorzugt Katalysatoren eingesetzt, die in reduzierter, aktiver Form das Edelmetall zumindest teilweise in der Oxidationsstufe 0 und Zink vorzugsweise in der Oxidationsstufe +2 enthalten.

Die Katalysatoren werden nach an sich bekannten Verfahren hergestellt, z. B. durch Fällen von Carbonaten, Imprägnieren vorgefertigter Träger oder durch Mischen von Precursor-Verbindungen, sowie durch nachfolgende Kalzinierung.

Die Katalysatoren werden zweckmäßig in eine Form gebracht, die bei der Hydrierung einen geringen Strömungswiderstand bietet, wie beispielsweise Tabletten, Zylinder, Strangextrudate oder Ringe.

Im erfindungsgemäßen Verfahren wird die Hydrierung bevorzugt in flüssiger Phase durchgeführt. Die Hydrierung kann an suspendierten oder stückigen im Festbett angeordneten Katalysatoren kontinuierlich oder diskontinuierlich durchgeführt werden. Im erfindungsgemäßen Verfahren wird eine kontinuierliche Hydrierung an einem im Festbett angeordnetem Katalysator, bei dem sich unter Reaktionsbedingungen die Produkt/Eduktphase hauptsächlich im flüssigen Zustand befindet, bevorzugt.

Wenn die Hydrierung kontinuierlich an einem im Festbett angeordneten Katalysator durchgeführt wird, ist es zweckmäßig, den Katalysator vor der Hydrierung in die aktive Form zu überführen. Dies kann durch Reduktion des Katalysators mit Wasserstoff-haltigen Gasen nach einem Temperaturprogramm erfolgen. Dabei kann die Reduktion gegebenenfalls in Gegenwart einer flüssigen Phase, die über den Katalysator rieselt, durchgeführt werden. Als flüssige Phase kann dabei ein Lösemittel oder das Hydrierprodukt eingesetzt werden.

Für das erfindungsgemäße Verfahren können unterschiedliche Verfahrensvarianten gewählt werden. Es kann adiabatisch, polytrop oder praktisch isotherm, d. h. mit einem Temperaturanstieg von typischerweise kleiner als 10 °C, ein- oder mehrstufig durchgeführt werden. Im letzteren Falle kann man alle Reaktoren, zweckmäßig Rohrreaktoren, adiabatisch oder praktisch isotherm sowie einen oder mehrere adiabatisch und die anderen praktisch isotherm betreiben. Weiterhin ist es möglich, die aromatischen Polycarbonsäureester im geraden Durchgang oder unter Produktrückführung zu hydrieren.

Das erfindungsgemäße Verfahren wird in der Flüssig/Gas-Mischphase oder Flüssigphase in Dreiphasenreaktoren im Gleichstrom durchgeführt, wobei das Hydriergas in an sich bekannter Weise im flüssigen Edukt/Produktstrom verteilt wird. Im Interesse einer gleichmäßigen Flüssigkeitsverteilung, einer verbesserten Reaktionswärmeabfuhr und einer hohen Raum-ZeitAusbeute werden die Reaktoren vorzugsweise mit hohen Flüssigkeitsbelastungen von 15 bis 120, insbesondere von 25 bis 80 m³ pro m² Querschnitt des leeren Reaktors und Stunde betrieben. Wird ein Reaktor im geraden Durchgang betrieben, so kann die spezifische Katalysatorbelastung (LHSV) Werte zwischen 0,1 und 10 h⁻¹ annehmen.

Die Hydrierung kann in Ab- oder vorzugsweise in Anwesenheit eines Lösungsmittels durchgeführt werden. Als Lösemittel können alle Flüssigkeiten eingesetzt werden, die mit dem Edukt und Produkt eine homogene Lösung bilden, sich unter Hydrierbedingungen inert verhalten und sich leicht vom Produkt abtrennen lassen. Das Lösemittel kann auch ein Gemisch mehrerer Stoffe sein und gegebenenfalls Wasser enthalten.

Beispielsweise können folgende Stoffe als Lösemittel eingesetzt werden:

Geradkettige oder cyclische Ether, wie beispielsweise Tetrahydrofuran oder Dioxan, sowie aliphatische Alkohole, in denen der Alkylrest 1 bis 13 Kohlenstoffatome aufweist.

Bevorzugt verwendbare Alkohole sind Isopropanol, n-Butanol, Isobutanol, n-Pentanol, 2-Ethylhexanol, Nonanole, technische Nonanolgemische, Decanol, technische Decanolgemische, Tridecanole.

Bei Einsatz von Alkoholen als Lösemittel kann es zweckmäßig sein, denjenigen Alkohol oder dasjenige Alkoholgemisch zu verwenden, das bei der Verseifung des Produkts entstehen würde. Dadurch würde die Nebenproduktbildung durch Umesterung ausgeschlossen. Ein weiteres bevorzugtes Lösemittel ist das Hydrierprodukt selbst.

Durch die Verwendung eines Lösemittels kann die Aromatenkonzentration im Reaktorzulauf begrenzt werden, wodurch eine bessere Temperaturkontrolle im Reaktor erreicht werden kann. Dies kann eine Minimierung von Nebenreaktionen und somit eine Erhöhung der Produktausbeute zur Folge haben. Bevorzugt liegt der Aromatengehalt im Reaktorzulauf zwischen 1 und 35 %, insbesondere zwischen 5 und 25 %. Der gewünschte Konzentrationsbereich kann bei Reaktoren, die in Schlaufenfahrweise betrieben werden, durch die Zirkulationsrate (Mengenverhältnis von rückgeführten Hydrieraustrag zu Edukt) eingestellt werden.

Das erfindungsgemäße Verfahren wird in einem Druckbereich von 3 bis 300 bar, insbesondere zwischen 15 und 200 bar, ganz besonders zwischen 50 und 200 bar durchgeführt. Die Hydriertemperaturen liegen zwischen 50 und 220, insbesondere zwischen 100 und 200 °C.

Als Hydriergase können beliebige Wasserstoff-haltige Gasgemische, die keine schädlichen Mengen an Katalysatorgiften wie beispielsweise Kohlenmonoxid oder Schwefelwasserstoff enthalten, eingesetzt werden. Die Inertgasbestandteile können beispielsweise Stickstoff oder Methan sein. Bevorzugt wird Wasserstoff in einer Reinheit von größer 95 %, insbesondere größer 98 % eingesetzt.

Nach dem erfindungsgemäßen Verfahren können aromatische Polycarbonsäuren oder deren Derivate, insbesondere deren Alkylester zu den entsprechenden alicyclischen Polycarbonsäureverbindungen umgesetzt werden. Dabei können sowohl Vollester als auch Partialester hydriert werden. Unter Vollester wird eine Verbindung verstanden, bei der alle Säuregruppen verestert sind. Partialester sind Verbindungen mit mindestens einer freien Säuregruppe (oder ggf. einer Anhydridgruppe) und mindestens einer Estergruppe.

Die nach dem erfindungsgemäßen Verfahren hergestellten Polycarbonsäureester enthalten bevorzugt 2, 3 oder 4 Esterfunktionen.

Die im erfindungsgemäßen Verfahren verwendeten Polycarbonsäureester sind bevorzugt Benzol-, Diphenyl-, Naphthalin- und/oder Anthracenpolycarbonsäureester. Die so erhaltenen alicyclischen Polycarbonsäureester bestehen aus einem oder mehreren, ggf. durch eine C-C-Bindung verknüpfte oder ankondensierte C₆-Ringe. Optional können auch Polycarbonsäureester mit einem Diphenyloxid-Grundgerüst eingesetzt werden.

Die Alkoholkomponente der Polycarbonsäureester besteht bevorzugt aus verzweigten oder unverzweigten Alkyl-, Cycloalkyl- oder Alkoxyalkylgruppen mit 1 - 25 Kohlenstoffatomen. Diese können in einem Molekül eines Polycarbonsäureesters gleich oder unterschiedlich sein, d. h. sie können gleiche oder verschiedene Isomeren oder Kettenlängen besitzen.

In einer bevorzugten Ausführungsform betrifft die vorliegende Erfindung ein Verfahren zur Hydrierung der 1,2-; 1,3- oder 1,4-Benzoldicarbonsäureester, und/oder der 1,2,3-; 1,2,4- oder 1,3,5-Benzoltricarbonsäureester, d. h. die erfindungsgemäßen Gemische enthalten die Isomere der 1,2-; 1,3- oder 1,4-Cyclohexandicarbonsäureester, oder der 1,2,3-; 1,3,5- oder 1,2,4-Cyclohexantricarbonsäureester.

Im erfindungsgemäßen Verfahren können Ester folgender aromatischer Carbonsäuren eingesetzt werden:
1,2-Naphthalindicarbonsäure, 1,3-Naphthalindicarbonsäure, 1,4-Naphthalindicarbonsäure, 1,5-Naphthalindicarbonsäure, 1,6-Naphthalindicarbonsäure, 1,7-Naphthalindicarbonsäure, 1,8-Naphthalindicarbonsäure, Phthalsäure (Benzol-1,2-dicarbonsäure), Isophthalsäure (Benzol-1,3-dicarbonsäure), Terephthalsäure (Benzol-1,4-dicarbonsäure), Benzol-1,2,3-tricarbonsäure, Benzol-1,2,4-tricarbonsäure (Trimellitsäure), Benzol-1,3,5-tricarbonsäure (Trimesinsäure), Benzol-1,2,3,4-tetracarbonsäure. Weiterhin können Säuren eingesetzt werden, die aus den genannten Säuren durch Substitution eines oder mehrerer am aromatischen Kem gebundenen Wasserstoffatome durch Alkyl-, Cycloalkyl- oder Alkoxyalkylgruppen entstehen.

Es ist möglich, Alkyl-, Cycloalkyl- sowie Alkoxyalkylester z. B. der oben genannten Säuren zu verwenden, wobei diese Reste unabhängig von einander 1 bis 25, insbesondere 3 bis 15, ganz besonders 8 bis 13 C-Atome, insbesondere 9 C-Atome, umfassen. Diese Reste können linear oder verzweigt sein. Hat ein Einsatzprodukt mehr als eine Estergruppe, dann können diese Reste gleich oder verschieden sein.

Im erfindungsgemäßen Verfahren können als Ester einer aromatischen Polycarbonsäure beispielsweise folgende Verbindungen eingesetzt werden:
Terephthalsäuremonomethylester, Terephthalsäuredimethylester, Terephthalsäurediethylester, Terephthalsäuredi-n-propylester, Terephthalsäuredibutylester, Terephthalsäuredüsobutylester, Terephthalsäuredi-tert.-butylester, Terephthalsäuremonoglykolester, Terephthalsäurediglykolester, Terephthalsäure-n-octylester, Terephthalsäuredüsooctylester, Terephthalsäuredi-2-ethyl-hexylester, Terephthalsäuredi-n-nonylester, Terephthalsäurediisononylester, Terephthalsäuredi-n-decylester, Terephthalsäuredi-n-undecylester, Terephthalsäurediisodecylester, Terephthalsäurediisododecylester, Terephthalsäure-ditridecylester, Terephthalsäuredi-n-octadecylester, Terephthalsäuredüsooctadecylester, Terephthalsäuredi-n-eicosylester, Terephthalsäure-monocyclohexylester; Phthalsäuremonomethylester, Phthalsäuredimethylester, Phthalsäuredi-n-propylester, Phthalsäuredi-n-butylester, Phthalsäuredüsobutylester, Phthalsäuredi-tert.-butylester, Phthalsäuremonoglykolester, Phthalsäurediglykolester, Phthalsäuredi-n-octylester, Phthalsäuredüsooctylester, Phthalsäuredi-2-ethylhexylester, Phthalsäuredi-n-nonylester, Phthalsäurediisononylester, Phthalsäuredi-n-decylester, Phthalsäuredi-2-propylheptylester, Phthalsäurediisodecylester, Phthalsäuredi-n-undecylester, Phthalsäurediisoundecylester, Phthalsäureditridecylester, Phthalsäuredi-n-octadecylester, Phthalsäurediisooctadecylester, Phthalsäuredi-n-eicosylester, Phthalsäuremonocyclohexylester; Phthalsäuredicyclohexylester, Isophthalsäuremonomethylester, Isophthalsäuredimethylester, Isophthalsäuredimethylester, Iso-phthalsäurediethylester, Isophthalsäuredi-n-propylester, Isophthalsäuredi-n-butylester, Iso-phthalsäuredüsobutylester, Isophthalsäuredi-tert.-butylester, Isophthalsäuremonoglykolester. Isophthalsäurediglykolester, Isophthalsäuredi-n-octylester, Isophthalsäurediisooctylester, Iso-phthalsäuredi-2-ethylhexylester, Isophthalsäuredi-n-nonylester, Isophthalsäurediisononylester, Isophthalsäuredi-n-decylester, Isophthalsäuredüsodecylester, Isophthalsäuredi-n-undecylester, Isophthalsäurediisododecylester, Isophthalsäuredi-n-dodecylester, Isophthalsäureditridecylester, Isophthalsäuredi-n-octadecylester, Isophthalsäurediisooctadecylester, Isophthalsäuredi-n-eicosylester, Isophthalsäuremonocyclohexylester.

Es können auch Gemische aus zwei oder mehreren Polycarbonsäureestern eingesetzt werden. Solche Gemische können beispielsweise auf folgenden Wegen erhalten werden:
a) eine Polycarbonsäure wird mit einem Alkohol derart partiell verestert, dass Voll- und Partialester nebeneinander vorliegen.
b) Ein Gemisch von mindestens zwei Polycarbonsäuren wird mit einem Alkohol verestert, wobei ein Gemisch von mindestens zwei Vollestern entsteht.
c) Eine Polycarbonsäure wird mit einem Alkoholgemisch versetzt, wobei ein Gemisch vieler Vollester entstehen kann.
d) Weiterhin kann eine Polycarbonsäure mit einem Alkoholgemisch partiell verestert werden.
e) Weiterhin kann ein Gemisch von mindestens zwei Carbonsäuren mit einem Alkoholgmisch partiell verestert werden.
f) Weiterhin kann ein Gemisch aus mindestens zwei Polycarbonsäuren mit einem Alkoholgemisch partiell verestert werden.

Bei den Umsetzungen a) bis f) können an Stelle der Polycarbonsäuren auch die entsprechenden Anhydride eingesetzt werden.

Großtechnisch werden aromatische Ester, insbesondere die Vollester auf Weg c) häufig aus Alkoholgemischen hergestellt.

Entsprechende Alkoholgemische sind beispielsweise:
C₅-Alkoholgemische, hergestellt aus linearen Butenen durch Hydroformylierung und anschließender Hydrierung;
C₅-Alkoholgemische, hergestellt aus Butengemischen, die lineare Butene und Isobuten enthalten, durch Hydroformylierung und anschließende Hydrierung;
C₆-Alkoholgemische, hergestellt aus einem Penten oder aus einem Gemisch von zwei oder mehreren Pentenen, durch Hydroformylierung und anschließende Hydrierung; C₇-Alkoholgemische, hergestellt aus Triethylen oder Dipropen oder einem Hexenisomer oder einem sonstigen Gemisch von Hexenisomeren, durch Hydroformylierung und anschließende Hydrierung;
C₈-Alkoholgemische, wie 2-Ethylhexanol (2 Isomere), hergestellt durch Aldolkondensation von n-Butyraldehyd und anschließende Hydrierung;
C₉-Alkoholgemische, hergestellt aus C₄-Olefinen durch Dimerisierung, Hydroformylierung und Hydrierung. Dabei kann zur Herstellung der C₉-Alkohole von Isobuten oder von einem Gemisch linearer Butene oder von Gemischen mit linearen Butenen und Isobuten ausgegangen werden. Die C₄-Olefine können mit Hilfe unterschiedlicher Katalysatoren dimerisiert werden, wie beispielsweise Protonensäuren, Zeolithe, metallorganische Nickelverbindungen oder feste nickelhaltige Kontakte. Die Hydroformylierung der C₈-Olefingemische kann mit Hilfe von Rhodium- oder Kobaltkatalysatoren erfolgen. Es gibt daher ein Vielzahl von technischen C₉-Alkoholgemischen.
C₁₀-Alkoholgemische, hergestellt aus Tripropylen durch Hydroformylierung und anschließende Hydrierung; 2-Propylheptanol (2 Isomere), hergestellt durch Aldolkondensation von Valeraldehyd und anschließende Hydrierung;
C₁₀-Alkoholgemische, hergestellt aus einem Gemisch von mindestens zwei C₅-Aldehyden durch Aldolkondensation und anschließende Hydrierung;
C₁₃-Alkolgemische, hergestellt aus Hexaethylen, Tetrapropylen oder Tributen durch Hydroformylierung und anschließende Hydrierung.

Weitere Alkoholgemische können durch Hydroformylierung und anschließende Hydrierung aus Olefinen bzw. Olefingemischen gewonnen werden, die beispielsweise bei Fischer-Tropsch-Synthesen, bei Dehydrierungen von Kohlenwasserstoffen, Metathesereaktionen, beim Polygasverfahren oder anderen technischen Prozessen anfallen.

Darüber hinaus können auch Olefingemische mit Olefinen unterschiedlicher C-Zahlen für die Herstellung von Alkoholgemischen eingesetzt werden.

Im erfindungsgemäßen Verfahren können alle Estergemische, hergestellt aus aromatischen Polycarbonsäuren und den obengenannten Alkoholgemischen, eingesetzt werden. Erfindungsgemäß werden bevorzugt Ester, hergestellt aus Phthalsäure oder Phthalsäureanhydrid und einem Gemisch isomerer Alkohole mit 6 bis 13 C-Atomen, eingesetzt.

Beispiele für technische Phthalate, die im erfindungsgemäßen Verfahren eingesetzt werden können, sind folgende Produkte mit den Handelsnamen:
Vestinol C (Di.n-butylphthalat) (CAS Nr.84-74-2); Vestinol IB (Di-i-butylphthalat) (CAS Nr. 84-69-5); Jayflex DINP (CAS Nr.68515-48-0); Jayflex DIDP (CAS Nr.68515-49-1); Palatinol 9P (68515-45-7), Vestinol 9 (CAS Nr. 28553-12-0); TOTM (CAS Nr. 3319-31-1); Linplast 68-TM , Palatinol N (CAS Nr. 28553-12-0); Jayflex DHP (CAS Nr. 68515-50-4); Jayflex DIOP (CAS Nr. 27554-26-3); Jayflex UDP (CAS Nr. 68515-47-9); Jayflex DIUP (CAS Nr. 85507-79-5); Jayflex DTDP (CAS Nr.68515-47-9); Jayflex L9P (CAS Nr. 68515-45-7); Jayflex L911P (CAS Nr. 68515-43-5); Jayflex L11P (CAS Nr. 3648-20-2); Witamol 110 (CAS Nr. 68515-51-5); Witamol 118 ( Di-n-C8-C10-alkylphthalat) (CAS Nr.71662-46-9); Unimoll BB (CAS Nr. 85-68-7); Linplast 1012 BP (CAS Nr. 90193-92-3); Linplast 13XP (CAS Nr.27253-26-5); Linplast 610P (CAS Nr. 68515-51-5); Linplast 68 FP (CAS Nr. 68648-93-1); Linplast 812 HP (CAS Nr. 70693-30-0); Palatinol AH (CAS Nr. 117-81-7); Palatinol 711 (CAS Nr. 68515-42-4); Palatinol 911 (CAS Nr. 68515-43-5); Palatinol 11 (CAS Nr. 3648-20-2);
Palatinol Z (CAS Nr.26761-40-0); Palatinol DIPP (CAS Nr. 84777-06-0); Jayflex 77 ( CAS Nr. 71888-89-6); Palatinol 10 P (CAS Nr. 533-54-0); Vestinol AH (CAS Nr. 117-81-7).

Im Folgenden wird auf die möglichen Stereoisomere des alicyclischen Systems Bezug genommen, wobei zwischen der trans- und cis-Form unterschieden wird. Beispielsweise sind bei den 1,2-Cyclohexandicarbonsäureester, wie bereits erwähnt, die trans Formen diejenigen Verbindungen, bei denen die Estergruppen entweder axial-axial (a, a) oder äquatorial-äquatorial (e, e) ausgerichtet sind; bei den cis-Verbindungen ist eine Estergruppe axial (a) und die andere äquatorial (e) ausgerichtet. Für andere alicyclische Polycarbonsäureester können, wie bereits ausgeführt, bei der Unterscheidung dieser beiden Formen andere Ausrichtungen gelten.

In besonderen Varianten des erfindungsgemäßen Verfahrens werden Phthalsäuredinonylester oder ein Gemisch aus isomeren Phthalsäuredinonylestern zu einem Gemisch von isomeren 1,2-Cyclohexandicarbonsäuredinonylester, mit einem Anteil des/der bezüglich der Stellung der Carboxylgruppen am Cyclohexanring in cis-Stellung vorliegenden Isomers von über 93 Mol-% hydriert.

Analog kann ebenso Phthalsäuredi(2-ethylhexyl)ester zu 1,2-Cyclohexandicarbonsäuredi(2-ethylhexyl)ester oder Phthalsäuredidecylester zu 1,2-Cyclohexandicarbonsäuredidecylester umgesetzt werden. Bezüglich der cis/trans-Isomeren gilt das für den Isononylester ausgeführte.

Die erfindungsgemäß hergestellten Gemische enthalten über 93 Mol-%, bezogen auf die gesamte Estermenge, cis-Verbindung(en). Bevorzugt enthalten die Gemische 94 - 100, 95 -100, 96 -100, 97 -100, 98 -100 oder 99 - 100 Mol-% des oder der cis-Isomere.

Ein weiterer Gegenstand der vorliegenden Erfindung ist die Verwendung der erfindungsgemäßen oder erfindungsgemäß hergestellten alicyclischen Polycarbonsäureester als Weichmacher in Kunststoffen. Bevorzugte Kunststoffe sind PVC, Homo- und Copolymere auf Basis von Ethylen, Propylen, Butadien, Vinylacetat, Glycidylacrylat, Glycidylmethacrylat, Acrylaten, Acrylaten mit am Sauerstoffatom der Estergruppe gebundenen Alkylresten von verzweigten oder unverzweigten Alkoholen mit einem bis zehn Kohlenstoffatome(n), Styrol, Acrylnitril, Homo- oder Copolymere von cyclischen Olefinen.

Als Vertreter der obigen Gruppen seien beispielsweise folgende Kunststoffe genannt: Polyacrylate mit gleichen oder verschiedenen Alkylresten mit 4 bis 8 C-Atomen, gebunden am Sauerstoffatom der Estergruppe, insbesondere mit dem n-Butyl-, n-Hexyl-, n-Octyl- und 2-Ethylhexylrest, Polymethacrylat, Polymethylmethacrylat, Methylacrylat-Butylacrylat-Copolymere, Methylmethacrylat-Butylmethacrylat-Copolymere, Ethylen-Vinylacetat-Copolymere, chloriertes Polyethylen, Nitrilkautschuk, Acrylnitril-Butadien-Styrol-Copolymere, Ethylen-Propylen-Copolymere, Ethylen-Propylen-Dien-Copolymere, Styrol-Acrylnitril-Copolymere, Acrylnitrl-Butadien-Kautschuk, Styrol-Butadien-Elastomere, Methylmethacrylat-Styrol-Butadien-Copolymere.

Darüber hinaus können die erfindungsgemäßen alicyclischen Polycarbonsäureester zur Modifizierung von Kunststoffmischungen, beispielsweise der Mischung eines Polyolefins mit einem Polyamid, eingesetzt werden.

Gemische aus Kunststoffen und den erfindungsgemäßen oder erfindungsgemäß hergestellten alicyclischen Polycarbonsäureestern sind ebenfalls Gegenstand der vorliegenden Erfindung. Geeignete Kunststoffe sind die bereits genannten Verbindungen. Solche Gemische enthalten bevorzugt mindestens 5 Gew.-%, besonders bevorzugt 20-80 Gew.-%, ganz besonders bevorzugt 30-70 Gew.-% der alicyclischen Polycarbonsäureester.

Gemische aus Kunststoffen, insbesondere PVC, die einen oder mehrere der erfindungsgemäßen alicyclischen Polycarbonsäureester enthalten, können beispielsweise in folgenden Produkten enthalten sein:

Gehäuse für Elektrogeräte, wie beispielsweise Küchengeräte, Computergehäuse, Gehäuse und Bauteile von Phono- und Fernsehgeräte, Rohrleitungen, Apparaten, Kabeln, Drahtummantelungen, Isolierbändern, Fensterprofilen, im Innenausbau, im Fahrzeug- und Möbelbau, Plastisolen, in Bodenbelägen, medizinische Artikel, Lebensmittelveipackungen, Dichtungen, Folien, Verbundfolien, Schallplatten, Kunstleder, Spielzeug, Verpackungsbehälter, Klebebandfolien, Bekleidung, Beschichtungen, Fasern für Gewebe.

Weiterhin können Gemische aus Kunststoff, insbesondere PVC, die einen oder mehrere der erfindungsgemäßen alicyclischen Polycarbonsäureester enthalten, beispielsweise zur Herstellung folgender Erzeugnisse verwendet werden:

Ein Gehäuse für Elektrogeräte, eine Rohrleitung, eine Vorrichtung, ein Kabel, eine Draht-Ummantelung, ein Fensterprofil, ein Bodenbelag, ein medizinischer Artikel, ein Spielzeug, eine Lebensmittelverpackung, eine Dichtung, eine Folie, eine Verbundfolie, eine Schallplatte, Kunstleder, ein Verpackungsbehälter, eine Klebebandfolie, Bekleidung, eine Beschichtung, oder eine Faser für Gewebe.

Neben den obengenannten Anwendungen können die erfindungsgemäßen alicyclischen Polycarbonsäureester als Schmierölkomponente, als Bestandteil von Kühlflüssigkeiten und Metallbearbeitungsflüssigkeiten verwendet werden.

Die folgenden Beispiele sollen die Erfindung erläutern, ohne den Schutzbereich, definiert durch die Patentansprüche, einzuschränken.

### Analytik:

Das Verhältnis von cis- und trans-1.2-Cyclohexancarbonsäurediester wurde durch ¹H-NMR-Spektoskopie bestimmt.
- Messgerät:: NMR-Spektrometer Avance DPX-360 der Firma Bruker
- Messfrequenz:: 360 MHz
- Probenkopf:: QNP-Probenkopf, 5mm
- Lösungsmittel:: CDCl₃ (Deuterierungsgrad 99,8 %)
- Standard:: Tetramethylsilan (TMS)
- Messtemperatur:: 303 K
- Anzahl Scans:: 32
- Delay:: 1 s
- Aquisitonszeit:: 4,4 s
- Spektrale Breite:: 7440,5 Hz
- Pulswinkel:: 30°
- Pulslänge:: 3,2 µs

Zur Aufnahme der ¹H-NMR-Spektren wurden z. B. ca. 20 mg der Probe in ca. 0,6 ml CDCl₃ (mit 1 Gew.-% TMS) gelöst. Die Spektren wurden unter den oben angegebenen Bedingungen aufgenommen und aus TMS = 0 ppm referenziert.

In den erhaltenen ¹H-NMR-Spektren ließen sich die Methinsignale der cis- und trans-Dialkylhexahydrophthalate mit den chemischen Verschiebungen von ca. 2,8 ppm bzw. 2,6 ppm unterscheiden. Dabei entsprach das zu tieferem Feld verschobene Signal (größerer ppm-Wert) der cis-Verbindung. Zur Quantifizierung der Isomere wurden die Integrale von 3,0 ppm bis 2,7(2) ppm und von 2,7(2) ppm bis 2,5 ppm bestimmt, wobei die Trennung der beiden Integrale in der Mittel zwischen den Signalen erfolgte. Aus den Intensitätsverhältnissen konnte das Verhältnis der beiden isomeren Strukturen bestimmt werden.

### Beispiel 1 (Vergleichsbeispiel)

Eingesetzt wurde der Katalysator H 14184 (0,5 % AG auf Übergangstonerde in Form von Strängen mit 1,2 mm Durchmesser) der Degussa. In einem Rührkessel-Autoklaven wurden 57 g Katalysator in einen rotierenden Korb gegeben und nach Herstellervorschrift in Wasserstoff bei 4 bar und 200° C reduziert. Anschliessend wurde der Autoklav mit 600 g Diisononylphthalat (kurz DINP, Produkt Vestinol 9 der Oxeno GmbH) aufgefüllt und mit Wasserstoff unter einem Druck von 200 bar beaufschlagt. Dann wurde bei einer Temperatur von 120°C 70 Stunden lang hydriert. Der Umsatz des DINP war vollständig. Im Produkt wurde eine Anteil an cis-Diisononyl-Cyclohexandicarboxylat (cis-DINCH) von 85 % gefunden; der Rest war trans-DINCH.

### Beispiel 2 (Vergleichsbeispiel)

Der Versuch gemäss Beispiel 1 wurde wiederholt, allerdings wurde hier die Reaktionstemperatur auf 200°C angehoben und die Hydrierzeit auf 21,2 h verkürzt. Der Umsatz war wiederum vollständig, der Gehalt an cis-DINCH betrug nur noch 81,4 %. Durch die sehr hohe Reaktionstemperatur wurde zwar eine hohe Reaktionsgeschwindigkeit erzielt, die Isomerenselektivität ging jedoch stark zurück.

### Beispiel 3 (erfindungsgemäss)

Eingesetzt wurde der Katalysator H 14184 (0,5 % Pd auf Übergangstonerde in Form von Strängen mit 1,2 mm Durchmesser) der Degussa AG, der zusätzlich mit 2 % ZnO dotiert worden war. In einem Rührkessel-Autoklaven wurden 59 g Katalysator in einen rotierenden Korb gegeben und nach Herstellervorschrift in Wasserstoff bei 4 bar und 200° C reduziert. Anschliessend wurde der Autoklav mit 600 g Diisononylphthalat (kurz DINP, Produkt Vestinol 9 der Oxeno GmbH) aufgefüllt und mit Wasserstoff unter einem Druck von 200 bar beaufschlagt. Dann wurde bei einer Temperatur von 120°C 60 Stunden lang hydriert. Der Umsatz des DINP war vollständig. Im Produkt wurde eine Anteil an cis-Diisononyl-Cyclohexandicarboxylat (cis-DINCH) von 97,2 % gefunden; der Rest war trans-DINCH.

### Beispiel 4 (Vergleichsbeispiel)

Eingesetzt wurde ein Katalysator mit 1,0 % Ru auf α-Al₂O₃ in Form von Kugeln mit 3 mm Durchmesser. In einem Rührkessel-Autoklaven wurden 74 g Katalysator in einen rotierenden Korb gegeben und nach Herstellervorschrift in Wasserstoff bei 4 bar und 200° C reduziert. Anschliessend wurde der Autoklav mit 600 g Diisononylphthalat (kurz DINP, Produkt Vestinol 9 der Oxeno GmbH) aufgefüllt und mit Wasserstoff unter einem Druck von 200 bar beaufschlagt. Dann wurde bei einer Temperatur von 120°C 22 Stunden lang hydriert. Der Umsatz des DINP war vollständig. Im Produkt wurde eine Anteil an cis-Diisononyl-Cyclohexandicarboxylat (cis-DINCH) von 93,6 % gefunden; der Rest war trans-DINCH.

### Beispiel 5 (erfindungsgemäss)

Der Versuch gemäss Beispiel 4 wurde unter gleichen Bedingungen wiederholt, allerdings wurde hier ein Katalysator eingesetzt, der zusätzlich mit 2,5 % ZnO dotiert worden war. Der Umsatz war wiederum vollständig, der Gehalt an cis-DINCH betrug 97,3 %.

## Patentansprüche

1. Verfahren zur Herstellung von alicyclischen Polycarbonsäureestergemischen, die mindestens zwei Isomere bezüglich der Stellung der Estergruppen am Ringsystem und die einen Anteil des cis-Isomers über 93 Mol-% aufweisen, durch katalytische Hydrierung der entsprechenden aromatischen Polycarbonsäureester,
**dadurch gekennzeichnet,**
**dass** der Katalysator mindestens ein Edelmetall aus der VIII. Nebengruppe und mindestens ein Metall der II. Nebengruppe des Periodensystems enthält.

2. Verfahren nach Anspruch 1,
**dadurch gekennzeichnet,**
**dass** die Hydrierung bei 50 bis 220 °C und 3 bis 300 bar durchgeführt wird.

3. Verfahren nach Anspruch 1 oder 2,
**dadurch gekennzeichnet,**
**dass** die aromatischen Polycarbonsäureester Benzol-, Diphenyl-, Diphenyloxid-, Naphthalin- oder Anthracenpolycarbonsäureester sind.

4. Verfahren nach einem der Ansprüche 1 bis 3,
**dadurch gekennzeichnet,**
**dass** die Polycarbonsäureester 2, 3 oder 4 Estergruppen enthalten.

5. Verfahren nach einem der Ansprüche 1 bis 4,
**dadurch gekennzeichnet,**
**dass** die Alkoholkomponenten der Polcarbonsäureester Alkoxyalkyl-, Cycloalkyl-, und/oder Alkylgruppen mit 1 bis 25 Kohlenstoffatomen, verzweigt oder unverzweigt, jeweils gleich oder unterschiedlich sind.

## Claims

1. Process for preparing alicyclic polycarboxylic ester mixtures which have at least two isomers with respect to the position of the ester groups on the ring system, and which have above 93 mol% proportion of the cis isomer, by catalytic hydrogenation of the corresponding aromatic polycarboxylic esters, **characterized in that**
the catalyst comprises at least one precious metal of the 8th transition group, and at least one metal of the 2nd transition group, of the Periodic Table.

2. Process according to Claim 1,
**characterized in that**
the hydrogenation is carried out at from 50 to 220°C and at from 3 to 300 bar.

3. Process according to Claim 1 or 2,
**characterized in that**
the aromatic polycarboxylic esters are benzene- or biphenylpolycarboxylic esters, diphenyl oxide polycarboxylic esters, or naphthalene- or anthracenepolycarboxylic esters.

4. Process according to any of Claims 1 to 3, **characterized in that**
the polycarboxylic esters contain 2, 3, or 4 ester groups.

5. Process according to any of Claims 1 to 4, **characterized in that**
the alcohol components of the polycarboxylic esters are alkoxyalkyl, cycloalkyl, and/or alkyl groups having from 1 to 25 carbon atoms, branched or unbranched, in each case identical or different.

## Revendications

1. Procédé de fabrication de mélanges d'esters d'acides polycarboxyliques alicycliques, comprenant au moins deux isomères au regard de la position des groupes ester sur le système cyclique et la proportion de l'isomère cis étant supérieure à 93 % en moles, par hydrogénation catalytique des esters d'acides polycarboxyliques aromatiques correspondants, **caractérisé en ce que**
le catalyseur contient au moins un métal noble du groupe de transition VIII et au moins un métal du groupe de transition II du tableau périodique.

2. Procédé selon la revendication 1, **caractérisé en ce que** l'hydrogénation est réalisée à une température de 50 à 220 °C et à une pression de 3 à 300 bar.

3. Procédé selon la revendication 1 ou 2, **caractérisé en ce que** les esters d'acides polycarboxyliques aromatiques sont des esters de l'acide benzène-, diphényl-, diphényloxyde-, naphtaline- ou anthracène-polycarboxylique.

4. Procédé selon l'une quelconque des revendications 1 à 3, **caractérisé en ce que** les esters d'acides polycarboxyliques contiennent 2, 3 ou 4 groupes ester.

5. Procédé selon l'une quelconque des revendications 1 à 4, **caractérisé en ce que** les composants alcool des esters d'acides polycarboxyliques sont des groupes alcoxyalkyle, cycloalkyle et/ou alkyle contenant 1 à 25 atomes de carbone, ramifiés ou non ramifiés, à chaque fois identiques ou différents.
